(19)

**Europäisches Patentamt**
**European Patent Office**
**Office européen des brevets**

(11) **EP 4 765 132 A1**

(12) **EUROPEAN PATENT APPLICATION**
published in accordance with Art. 153(4) EPC

(43) Date of publication:
24.06.2026 Bulletin 2026/26

(21) Application number: 24854390.2

(22) Date of filing: 07.08.2024

(51) International Patent Classification (IPC):
$G16B\ 40/20^{(2019.01)}$      $G16B\ 30/00^{(2019.01)}$
$G16B\ 15/30^{(2019.01)}$      $G16B\ 20/20^{(2019.01)}$
$G16B\ 20/50^{(2019.01)}$      $G06N\ 3/0464^{(2023.01)}$
$G06N\ 3/09^{(2023.01)}$       $G06N\ 20/20^{(2019.01)}$

(52) Cooperative Patent Classification (CPC):
G06N 3/0464; G06N 3/09; G06N 20/20;
G16B 15/30; G16B 20/20; G16B 20/50;
G16B 30/00; G16B 40/20

(86) International application number:
PCT/KR2024/011714

(87) International publication number:
WO 2025/037838 (20.02.2025 Gazette 2025/08)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 16.08.2023  KR 20230107065

(71) Applicant: **Korea Advanced Institute of Science
and Technology**
**Daejeon 34141 (KR)**

(72) Inventors:
• **CHOI, Jung Kyoon**
  **Sejong 30098 (KR)**
• **KIM, Jeong Yeon**
  **Daejeon 34127 (KR)**

(74) Representative: **Cabinet Becker et Associés**
**25, rue Louis le Grand**
**75002 Paris (FR)**

(54) **METHOD FOR DESIGNING PERSONALIZED CANCER VACCINE USING B CELL-REACTIVE
NEOANTIGEN**

(57)    The present disclosure relates to a method for designing a personalized cancer vaccine, using a B cell-reactive neoantigen, the method including the steps of: receiving a peptide sequence extracted from cancer tissue and allelic sequence data of B cell receptors; calculating the preferential binding strength between amino acids in the peptide sequence and allelic sequences of B cell receptors; and calculating B cell epitope prediction scores by inputting the preferential binding strength between amino acids to a trained neural network model.

**(Cont. next page)**

EP 4 765 132 A1

【FIG 1A】

**Description**

**[Technical Field]**

The present disclosure relates to a method for designing a personalized cancer vaccine using a B cell-reactive neoantigen and a computer program stored in a recording medium.

**[Background Art]**

Cancer is one of the highest causes of death worldwide, and in order to treat various cancers, various therapies, including resection surgery, chemotherapy, and the like, have been implemented in clinical trials to treat various types of cancer. Among them, an immunotherapy is a therapy that suppresses cancer by reactivating immune cells in the human body, and the efficacy thereof has been verified in various clinical trials. Specifically, a cancer immunotherapy using immune checkpoint blockades treats inflammatory cancer infiltrated by primed cancer-reactive T cells.

Cancer vaccines treat cancer by newly introducing antigens targeting cancer-causing viruses or cancer cells and generating primed cancer-reactive T cells, and are emerging as a key technology for the cancer immunotherapy through a combined therapy with immune checkpoint blockades. An important factor in the development stage of the cancer vaccines relates to the determination of an antigen to be used as the cancer vaccine, and the antigen is determined when a cancer-derived mutation is identified as a neoantigen through genome analysis of an individual cancer patient. When the corresponding neoantigen is commonly found in many cancer patients, the neoantigen may be developed as a universal cancer vaccine, and when the neoantigen is found only in specific patients or specific cancer cells, the neoantigen may be developed as a personalized cancer vaccine.

Recently, machine learning-based algorithms have been used to discover suitable neoantigens with immunogenicity, and attempts have been made to increase accuracy by including binding affinity with MHC class I and II, T cell receptor (TCR) binding affinity, and steps such as antigen processing in a prediction model.

Meanwhile, anti-tumor immunity has mainly been studied based on T cells, but recently, tertiary lymphoid structures (TLS) have been discovered and thus, have received attention for important and diverse roles of B cells. Within the TLS, B cells function to activate T cells through antigen recognition and interaction. Recently, it has been demonstrated that B cell-specific checkpoint molecules regulate tumor-specific effector T cell activity through antigen presentation and co-stimulation. Further, it has been demonstrated in a mouse cancer model that a specific neoantigen may activate tumor-specific B cells and CD4+ T cells to generate follicular helper T cells producing IL21, but it is not clearly found whether B cells generally contribute to neoantigen-induced immune responses in various types of human cancers. This is because reliable methods for identifying CD4+ T cell and B cell-reactive neoepitopes have not been developed.

**[Disclosure]**

**[Technical Problem]**

An object of the present disclosure is to provide a method for designing a personalized cancer vaccine using a B cell-reactive neoantigen and a computer program stored in a recording medium.

However, the objects of the present disclosure are not limited to the above-mentioned objects, and other objects not mentioned can be clearly understood by those skilled in the art from the following description.

**[Technical Solution]**

According to an embodiment of the present disclosure, a method for designing a personalized cancer vaccine may include the steps of: receiving a peptide sequence extracted from cancer tissue and allelic sequence data of B cell receptors; calculating the preferential binding strength between amino acids in the peptide sequence and allelic sequences of the B cell receptors; and calculating B cell epitope prediction scores by inputting the preferential binding strength between the amino acids to a trained neural network model.

According to an embodiment of the present disclosure, the step of calculating the preferential binding strength between amino acids in the peptide sequence and the allelic sequences of the B cell receptors may include a step of generating a matrix including $C\alpha$-$C\alpha$ distance information between amino acids and interatomic contact information between amino acid residues in the peptide sequence and the allelic sequences of the B cell receptors.

According to an embodiment of the present disclosure, the matrix may include somatic hypermutation (SHM) frequency data per residue of allelic superfamily of the B cell receptors.

According to an embodiment of the present disclosure, the cell values constituting the matrix may be defined by the following Equation 1:

[Equation 1]

$$P_{ij} = \left(I_{ij}\right) \times \left(1 + SHM_i\right),$$

Here, i and j may represent the positions of an immunoglobulin heavy chain variant (IGHV) of the B cell receptor and a peptide sequence, and $I_{ij}$ may represent a preferential binding interaction between $IGHV_i$ and $peptide_j$.

According to an embodiment of the present disclosure, the neural network model may be a convolutional neural network (CNN) model, and the CNN model may include a convolutional layer that detects spatial interactions between the immunoglobulin heavy chain variant (IGHV) of the B cell receptor and the peptide sequence.

The convolutional layer may output a feature map through a convolution operation on the input image. At this time, a filter that performs the convolution operation may also be called a kernel. The size of the filter may be referred to as a filter size or kernel size. The operational parameters constituting the kernel may be referred to as kernel parameters, filter parameters, or weights.

According to an embodiment of the present disclosure, the CNN model may consist of a plurality of convolutional layers, a fully connected layer, and a sigmoid layer.

According to an embodiment of the present disclosure, the kernel of the CNN model may include the entire amino acid sequence before complementarity determining region 3 (CDR3) of the immunoglobulin heavy chain variant (IGHV) of the B cell receptor.

According to an embodiment of the present disclosure, the convolutional layer may extract features of contact and distal interactions using a pooling-free rectified linear unit (ReLU) function.

According to an embodiment of the present disclosure, the neural network model may be an ensemble model that aggregates a plurality of CNN models.

According to an embodiment of the present disclosure, the step of calculating the B cell epitope prediction scores may include a step of applying linear regression to the output values of the neural network model.

According to an embodiment of the present disclosure, a neoantigen with the calculated B cell epitope prediction scores exceeding a predetermined reference value may be determined to have a high ability to induce an immune response. Here, the predetermined reference value may be an average B cell epitope prediction score, but is not limited thereto.

According to another embodiment of the present disclosure, there is provided a computer program stored in a computer-readable recording medium for executing on a computer the method for designing the personalized cancer vaccine using the B cell-reactive neoantigen in combination with hardware.

According to yet another embodiment of the present disclosure, there is provided a computer-readable recording medium recording programs for executing on a computer the method for designing the personalized cancer vaccine using the B cell-reactive neoantigen.

[Advantageous Effects]

According to the embodiment of the present disclosure, the method can be used to design a personalized cancer vaccine by calculating the binding strength of a B cell receptor and a B cell epitope to predict a neoantigen inducing a strong immune response with high accuracy.

According to the embodiment of the present disclosure, the method can predict a neoantigen that strongly induces a T cell immune response of a cancer vaccine, and thus can design a cancer vaccine based on a neoantigen with better performance.

It should be understood that the effects of the present disclosure are not limited to the effects described above, but include all effects that can be deduced from the detailed description of the present disclosure or configurations of the disclosure described in claims.

[Description of Drawings]

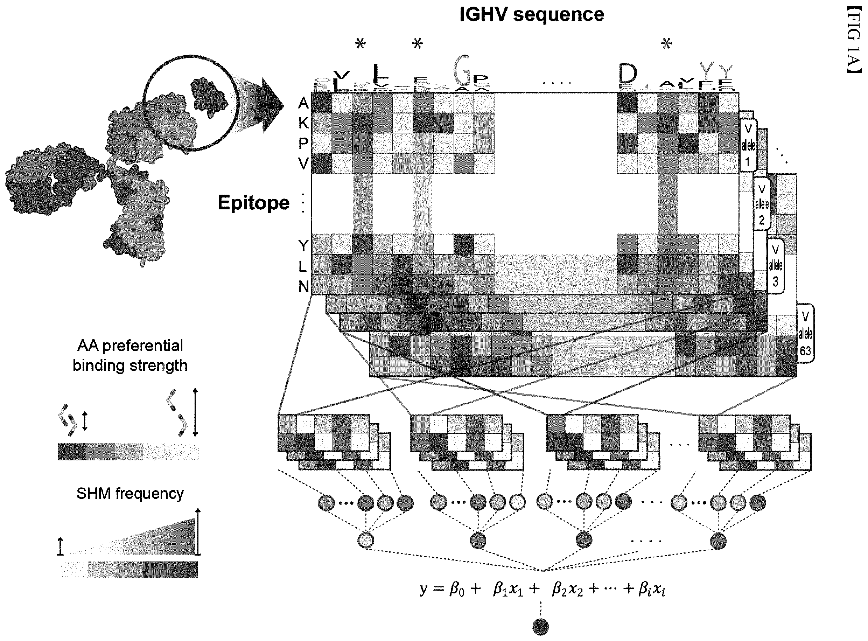

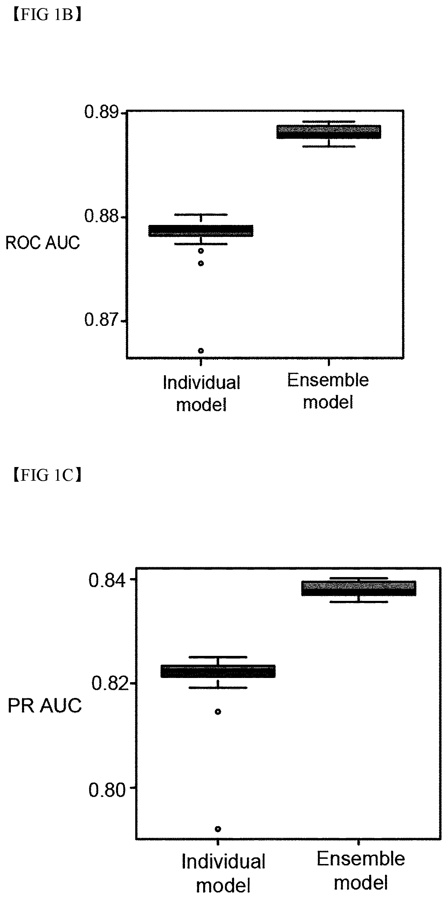

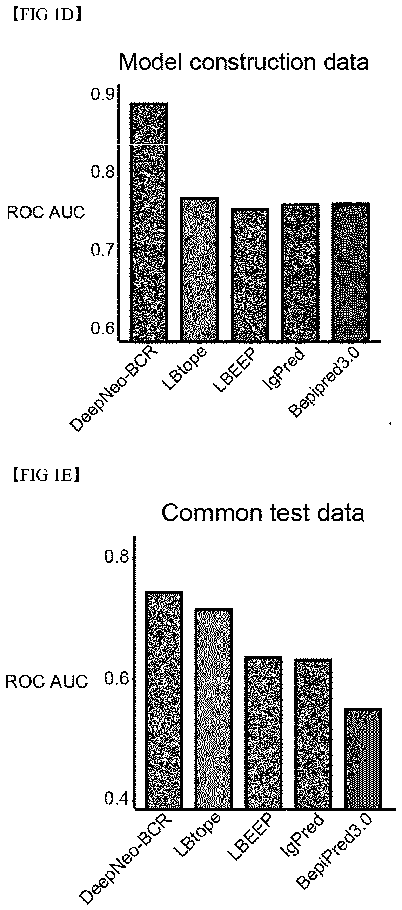

FIG. 1 shows the construction and evaluation of a B-cell epitope prediction model: (A) a schematic diagram of a B-cell epitope prediction model that identifies B-cell epitopes based on a CNN architecture; (B) comparison of the area under the receiver operating characteristic curve (ROC-AUC) between an individual allelic model and an ensemble model;

(C) comparison of the area under the precision-recall curve (PR-AUC) between an individual allelic model and an ensemble model (statistics are calculated using Welch two sample t test); (D) comparison of ROC-AUC values between a B-cell epitope prediction model (DeepNeo-BCR) and conventional linear B-cell epitope prediction tools; and (E) comparison of the ROC-AUC values between a B-cell epitope prediction model (DeepNeo-BCR) and conventional linear B-cell epitope prediction tools using a shared test dataset (IEDB items that had not been used for model construction are used).

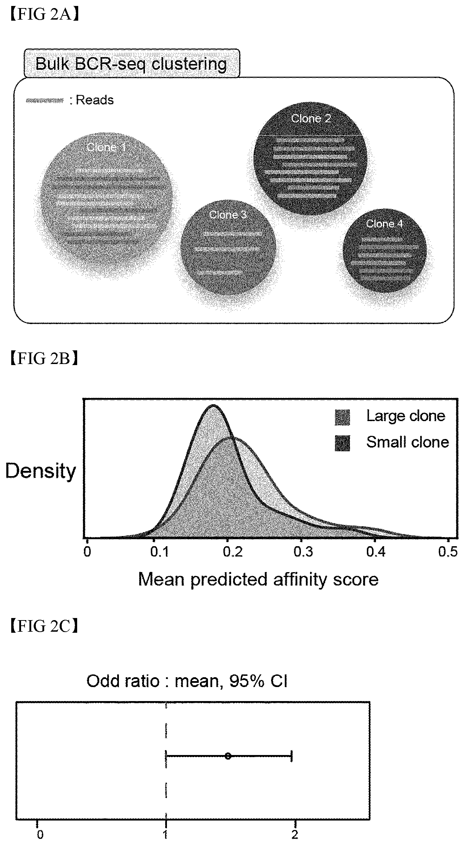

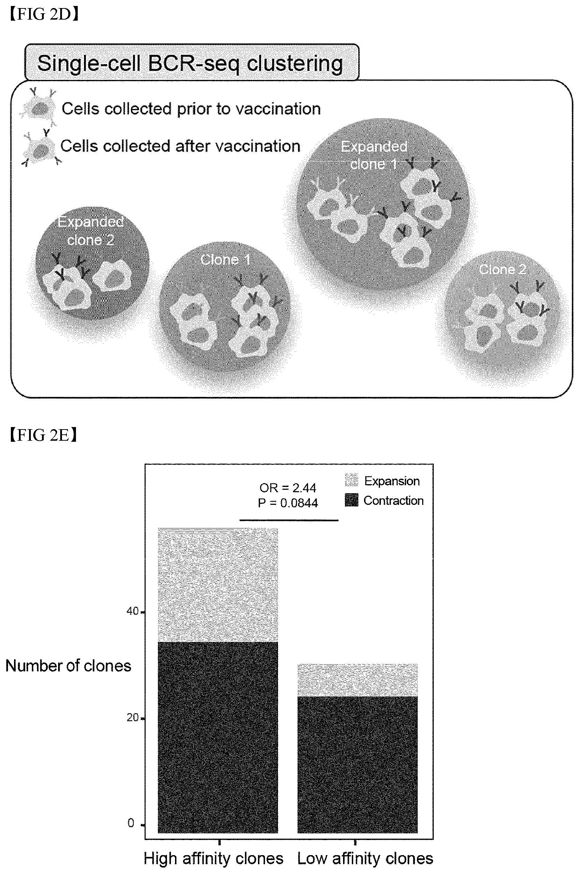

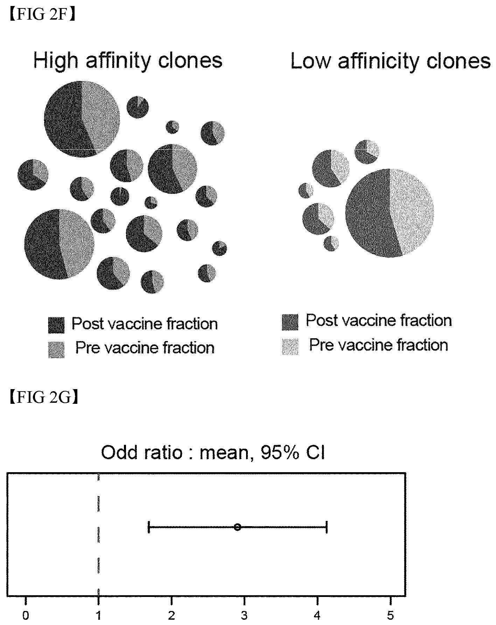

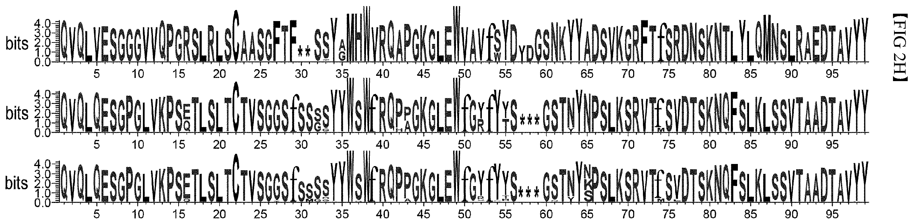

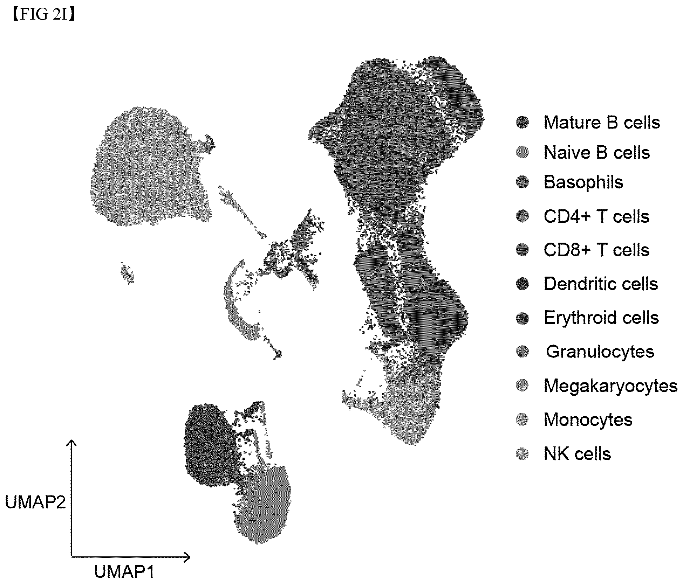

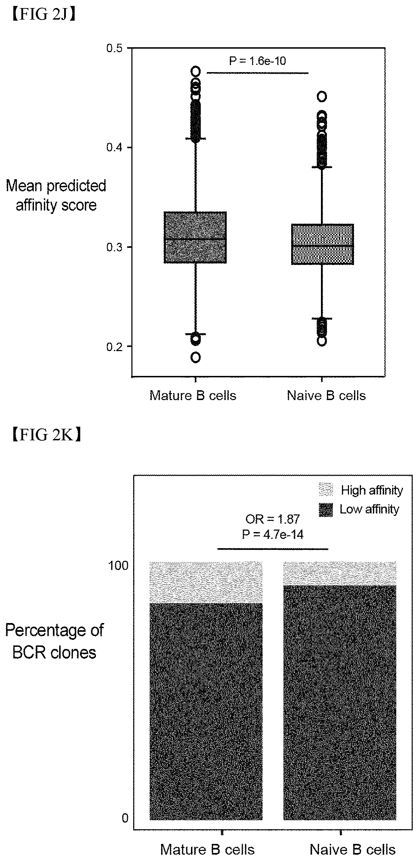

FIG. 2 shows validation of SARS-CoV-2 vaccine data: (A) a schematic diagram of clustering of bulk BCR sequencing data; (B) a distribution diagram of prediction scores of B-cell epitope prediction models for large clones (> mean read fraction) and small clones (< mean read fraction) after Pfizer SARS-CoV-2 vaccination in a sample (ID: CME93) (D = 0.32, P = 0.049 in Kolmogorov-Smirnov test); (C) odd ratios of high-scoring BCR clones in a large clonal group per patient; (D) a schematic diagram of clustering of single-cell BCR sequencing data (pre- and post-vaccination BCR sequences of each patient sample are clustered by sequence similarity); (E) odd ratios of high-scoring BCR clones through clonal expansion determined by a difference in cell fractions before and after vaccination (odd ratio P values are calculated using Fisher's exact test); (F) a diagram of clones expanded into larger (> 24 cells) clones based on predicted affinity scores calculated by the B-cell epitope prediction model; (G) odd ratios of high-scoring BCR clones in a large (> mean cell fraction) clone group using post-vaccination data (odd ratio P values are calculated using Fisher's exact test); (H) sequences of three expanded clones with the highest B-cell epitope prediction scores, (I) cell annotation for 76,843 cells in 24 samples after vaccination; (J) differences in mean B-cell epitope prediction scores between mature B cells and naive B cells (calculated using Welch two sample t-test); and (K) odd ratios of high-scoring BCR clones in a mature B-cell group (odd ratio P values are calculated using Fisher's exact test).

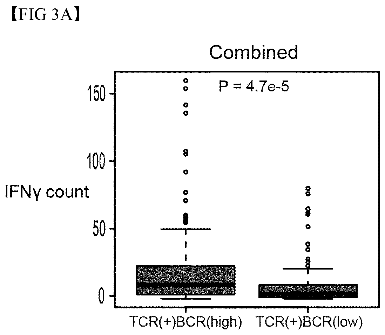

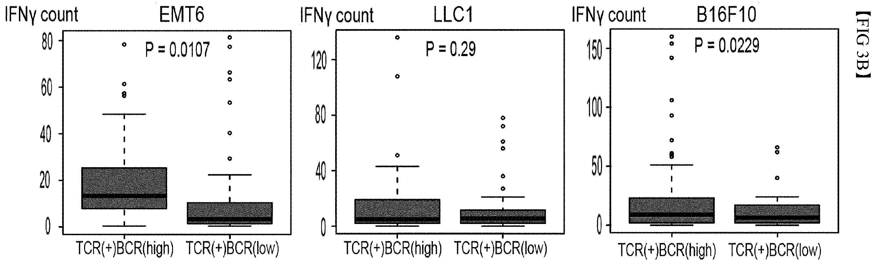

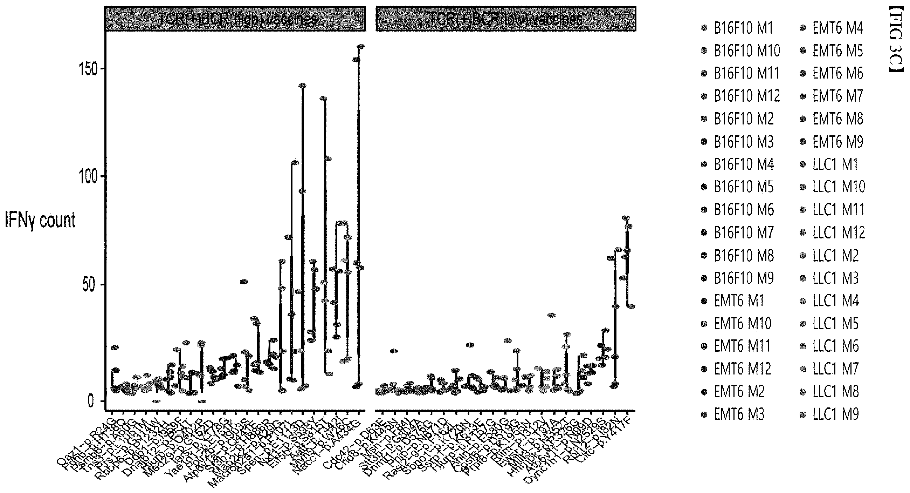

FIG. 3 shows effects of neoantigen vaccination on B cell reactivity in mice: (A) differences in IFNγ signaling between TCR(+)BCR(high) vaccines and TCR(+)BCR(low) vaccines (peptide n = 44 and ELISpot assay n = 264) (assigned to BCR-high and -low groups based on the maximum difference between mutant and wild-type B cell epitope prediction scores for each potential neoepitope); (B) differences in IFNγ signaling between TCR(+)BCR(high) vaccines and TCR(+)BCR(low) vaccines for EMT6 breast cancer, LLC1 lung cancer, and B16F10 melanoma models; and (C) comparison of individual ELISpot results between TCR(+)BCR(high) vaccines and TCR(+)BCR(low) vaccines (respective dots represent individual mice tested with the vaccines).

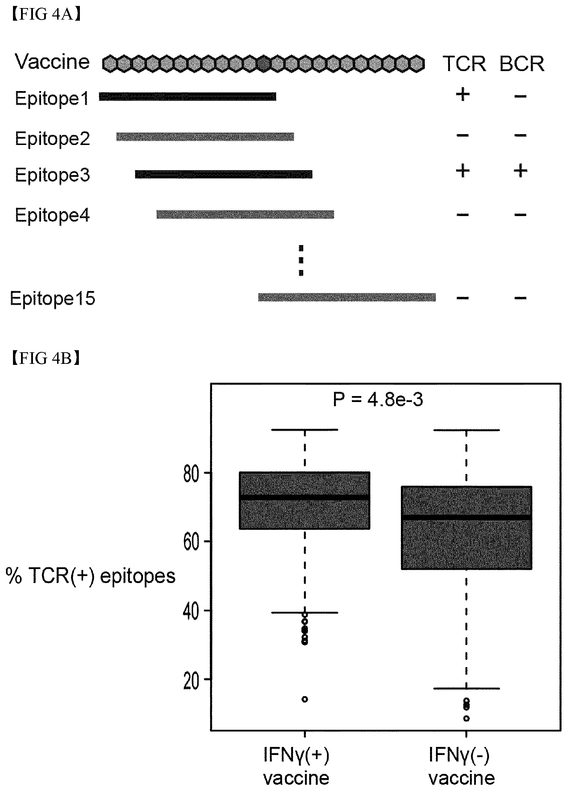

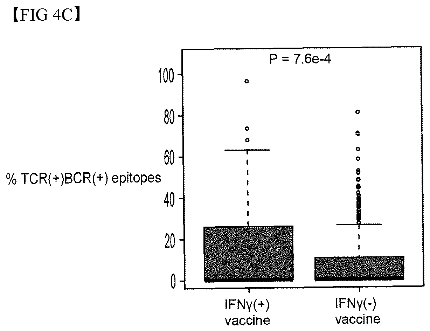

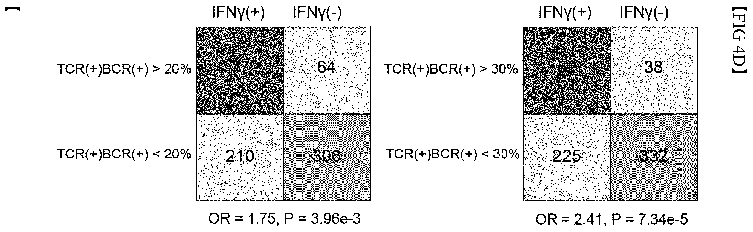

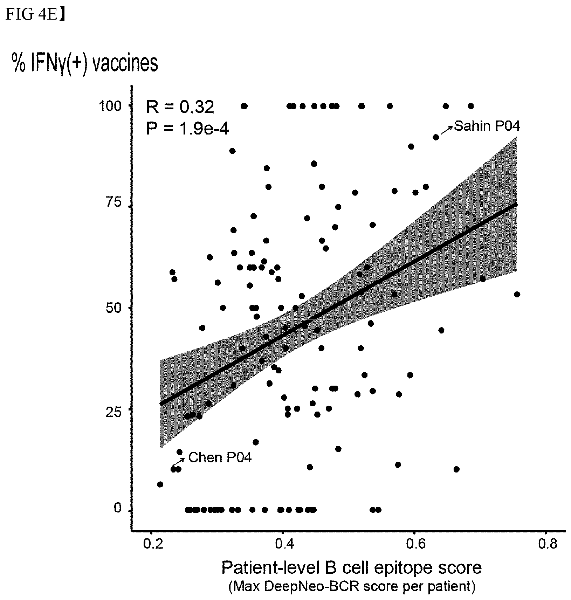

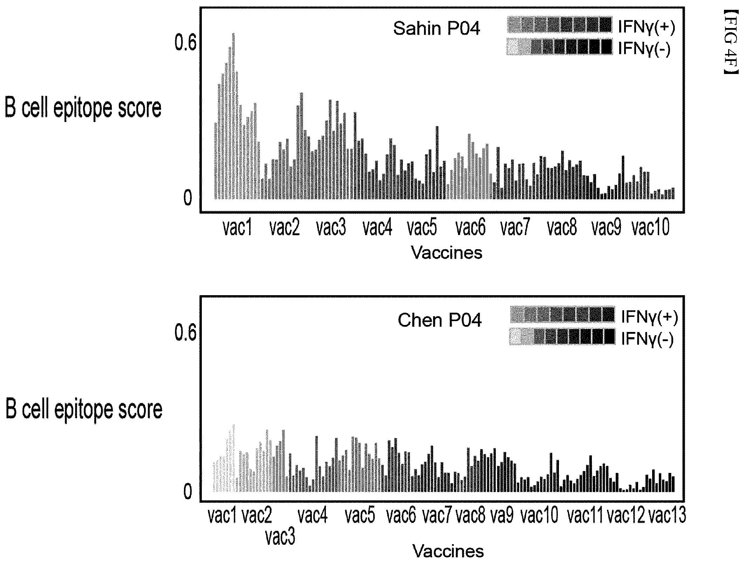

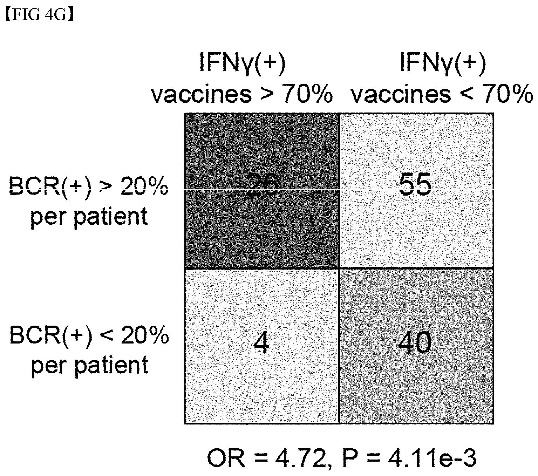

FIG. 4 shows effects of B cell reactivity in human personalized neoantigen vaccination: (A) a schematic diagram showing vaccines with somatic mutations, potential neoepitopes, and predicted TCR/BCR reactivity marked in red by searching a plurality of 15-mer peptides in a vaccine sequence and testing T cell and B cell reactivity using DeepNeo-TCR and B-cell epitope prediction models, respectively; (B) effects of TCR(+) epitopes in immune response induction confirmed by comparing percentages of neoepitopes predicted to stimulate CD4+ or CD8+ T cell responses normalized by a vaccine length between IFNγ(+) and IFNγ(-) vaccines; (C) effects of TCR(+)BCR(+) neoepitopes in immune response stimulation confirmed by comparing percentages of neoepitopes predicted to stimulate both T cells and B cells normalized by a vaccine length between IFNγ(+) and IFNγ(-) vaccines; (D) ratios (Odds) of IFNγ(+) vaccines with high TCR(+)BCR(+) ratios (left: > 20%, right: > 30%) (odd ratio P-values are calculated by Fisher's exact test); (E) a correlation between patient-level B-cell epitope prediction scores and responses to vaccines (highest B-cell epitope prediction scores per patient and patient-level immune responses (% of IFNγ(+) vaccine) are performed in all neoepitopes using Pearson correlation test); (F) examples of patient cases (top) with high immune response rate and patient cases (bottom) with low immune response rate (B-cell epitope prediction scores for potential neoepitopes derived from each vaccine are indicated, in which a red palette represents IFNγ(+) vaccines and a blue palette represents IFNγ(-) vaccines); and (G) ratios (Odds) of patients receiving a high ratio (> 20% BCR(+) epitopes) of B-cell reactive neoepitopes showing robust immune responses (> 70% of delivered vaccines) (odd ratio P values are calculated using Fisher's exact test).

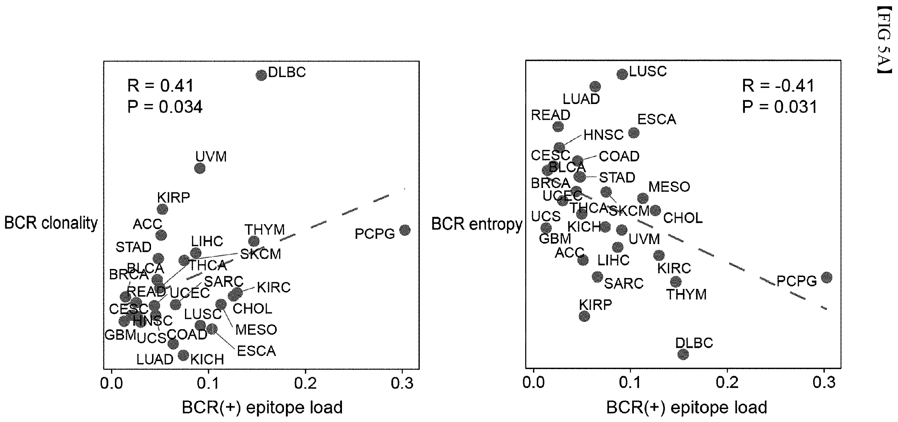

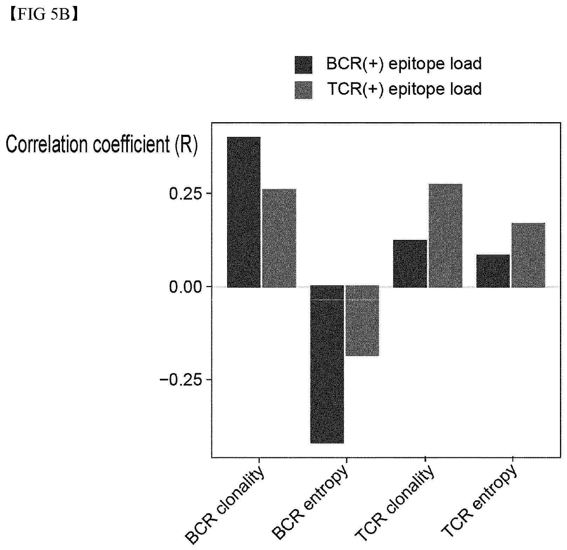

FIG. 5 shows correlations between B cell neoepitope load and tumor immune repertoire features: (A) a correlation between B cell neoepitope load averaged for cancer types and BCR features (clonality and entropy) in TCGA pan-cancer samples (n = 4,435); and (B) a correlation between B cell or T cell neoepitope load and BCR or TCR features (clonality and entropy) for cancer type (TCGA samples with MHC genotype information are used (n = 3,392)).

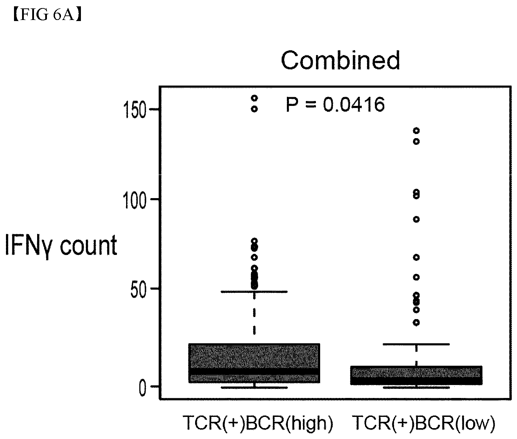

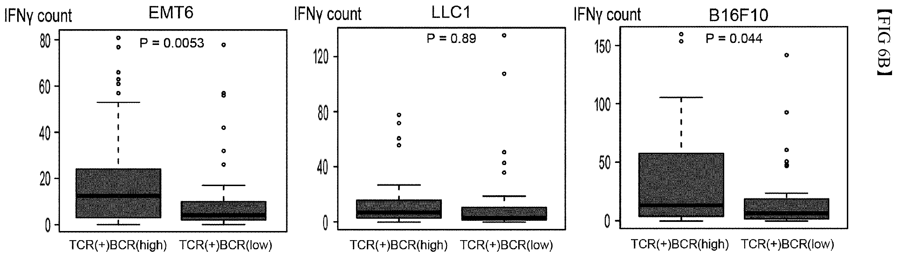

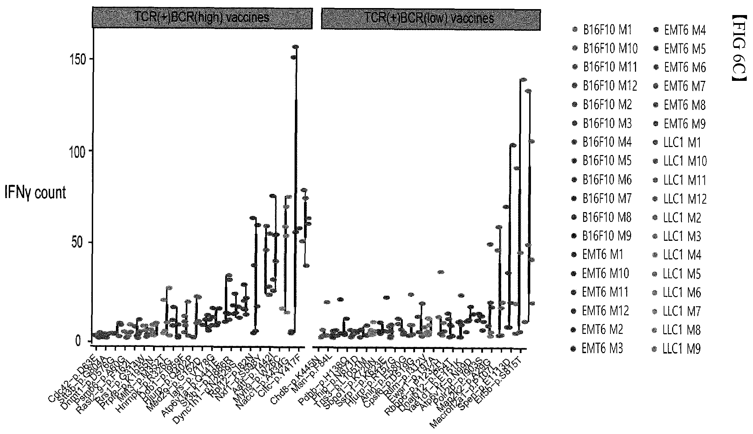

FIG. 6 shows effects of B cell reactivity in mouse neoantigen vaccination by repeating the same analysis as in FIG. 2 except that BCR-high and -low groups are classified by mutant B cell epitope prediction scores without considering wild-type B cell epitope prediction scores. The maximum score for all potential neoepitopes of each vaccine was retrieved and used for classification.

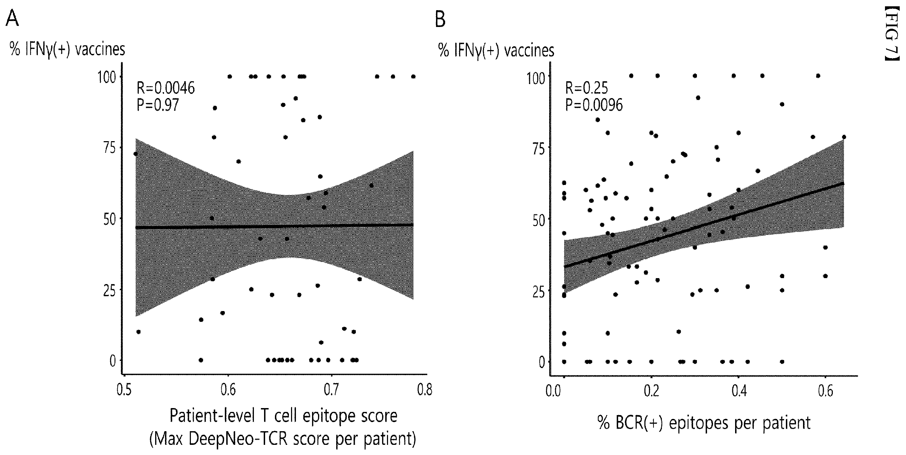

FIG. 7 shows results of patient-level immune response analysis: (A) a correlation between patient-level DeepNeo-TCR scores and responses to vaccines using DeepNeo-TCR for both CD8+ and CD4+ epitopes (the highest DeepNeo-TCR score among all neoepitopes per patient and the patient-level immune response (% of IFNγ(+) vaccine) is subjected to Pearson correlation test); and (B) a correlation between patient-level BCR(+) epitope amounts and a response rate to vaccines (an x-axis represents a percentage of predicted B cell binding epitopes among all putative peptides derived from all vaccines delivered to each patient).

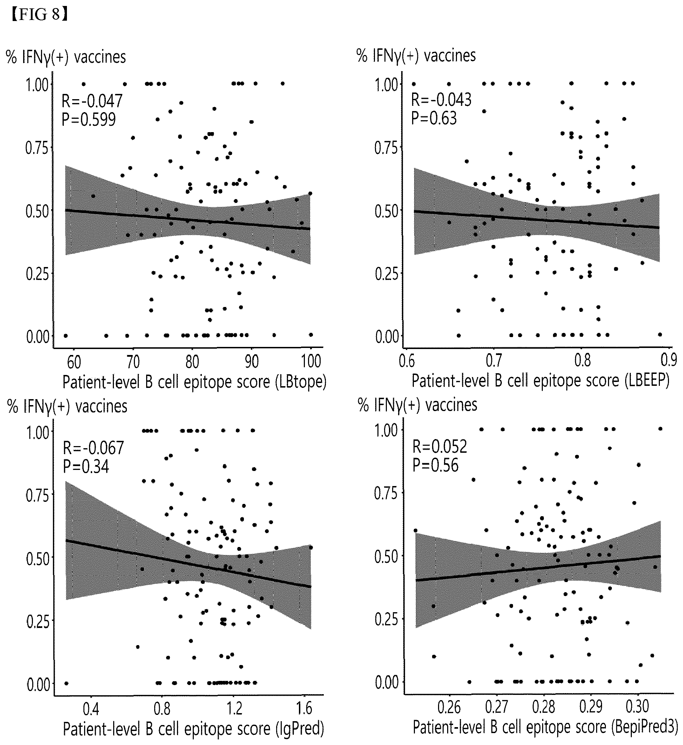

FIG. 8 shows patient-level immune response analysis using different epitope prediction tools by calculating a correlation between patient-level B cell epitope scores for all epitopes that may be derived from a vaccine sequence using LBtope, LBEEP, IgPred and BepiPred3.0. The maximum B-cell epitope score of the patient was used as a patient-level B-cell epitope score.

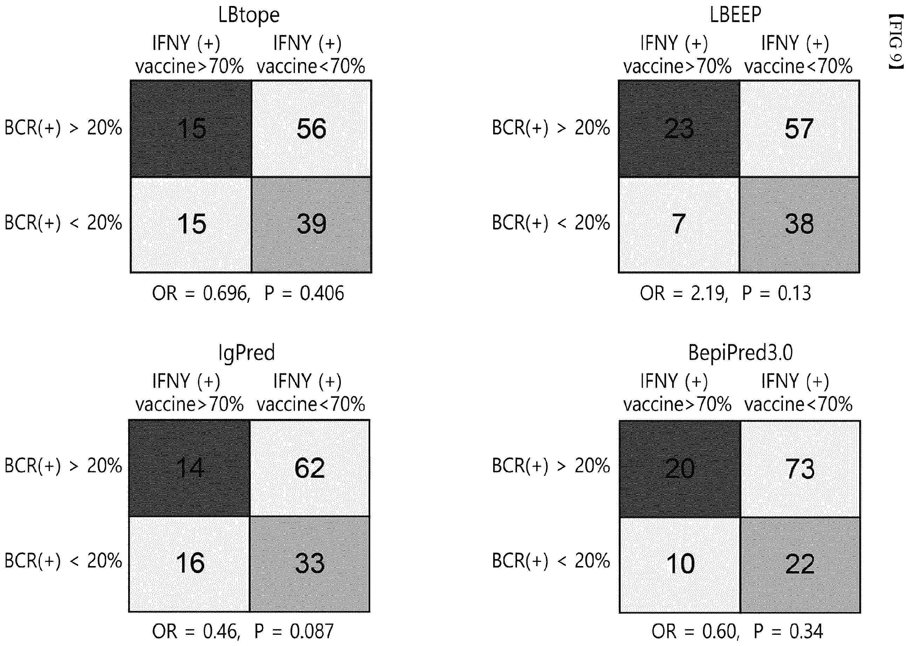

FIG. 9 shows additional patient-level immune response analysis using different epitope prediction tools, in which LBtope, LBEEP, IgPred and BepiPred3.0 were used to calculate the probability of patients receiving a high ratio of predicted neoepitopes (> 20% BCR(+) epitopes) that exhibit a strong immune response (> 70% of delivered vaccine). LBtope, IgPred, and BepiPred3.0 had no expected effect, and LBEEP showed limited statistical power with OR = 2.19 (P = 0.13) (odd ratio P values are calculated using Fisher's exact test).

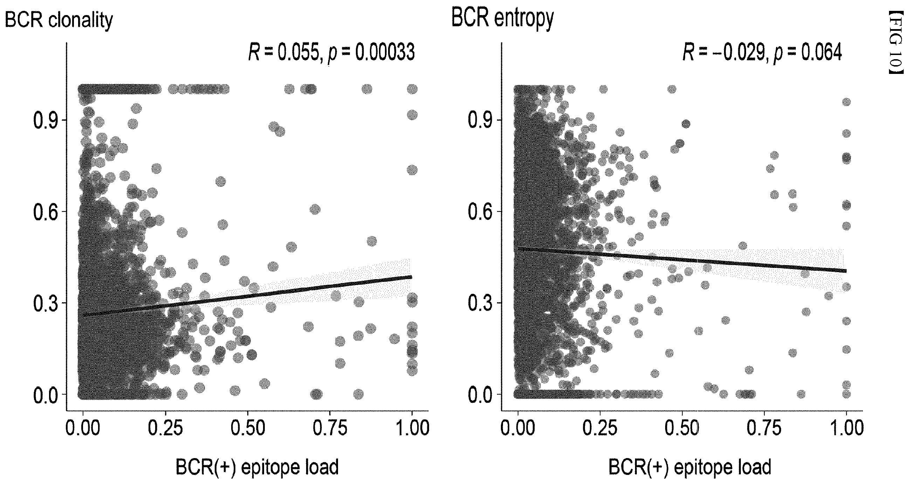

FIG. 10 shows sample-specific correlations between B cell neoepitope load and immune repertoire conditions: (A) a correlation between BCR(+) epitope load and BCR clonality for samples with matched mutation and immune repertoire data (n = 4,214); and (B) a correlation between BCR(+) epitope load and BCR entropy using individual samples (correlation coefficients are calculated using Pearson correlation test).

**[Best Mode]**

Hereinafter, embodiments will be described in detail with reference to the accompanying drawings. However, since various modifications may be made to embodiments, the scope of the present disclosure is not limited or restricted by these embodiments. It should be understood that all modifications, equivalents and substitutes for embodiments are included in the scope of the present disclosure.

The terms used in the embodiments are used for the purpose of description only, and should not be construed to be limited. A singular expression includes a plural expression unless the context clearly indicates otherwise. In the present specification, it should be understood that term "comprising" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as an ideal meaning or excessively formal meanings unless clearly defined in the present application.

As used herein, the term 'neoantigen' refers to a completely novel protein formed in cancer cells when a specific mutation occurs in tumor DNA, and may appear as an epitope on the cancer cells. The neoantigen is mainly caused by a non-synonymous mutation, and unlike tumor-associated antigens (TAAs) or cancer-germline antigens (CGAs) that are also expressed in some normal cells, the neoantigen is expressed only in cancer cells. The terms 'epitope,' 'antigenic peptide,' 'immunogenic peptide' and 'peptide' may be used interchangeably.

As used herein, the term 'neoepitope' refers to an epitope found in diseased cells, such as cancer cells.

As used herein, the term 'immunogenicity' refers to the ability to induce an immune response *in vivo,* and refers to the ability to induce an immune response associated with a therapy in cancer treatment.

As used herein, the term 'peptide' means an amino acid polymer, and may refer to an amino acid polymer or amino acid sequence expressed on the surface of cancer cells.

As used herein, the term 'exome' refers to a subset of the genome encoding a protein. The exome may refer to a collection of exons present in a cell, a cell group, or a subject.

As used herein, 'DeepNeo-BCR' refers to a cancer vaccine personalized design model using B cell epitope prediction that performs a method according to an embodiment of the present disclosure.

**[Modes]**

In describing the embodiments, a detailed description of related known technologies will be omitted if it is determined that they unnecessarily make the gist of the embodiments unclear.

Hereinafter, preferred Examples are presented in order to assist the understanding of the present disclosure. However, the following Examples are just provided to more easily understand the present disclosure and the contents of the present disclosure are not limited by Examples.

**Experimental Example 1: Data construction**

A list of linear epitopes before January 17, 2023 was collected from the immune epitope database (IEDB) through *in vitro* binding tests. The collected data was applied with several filters: (1) the peptides were required to be of desired lengths (12, 15, or 16-mer), (2) B cell and peptide binding experiments were required to be tested in humans or mice, (3) the peptides were not structural but linear, and (4) the peptides were required to be tested in IgG. After applying the filters, a total of 437,396 data were input by measuring B cell and peptide binding by qualitative measurement, cytokine secretion, direct measurement, or X-ray crystallography-based binding assay. Since experimental results may vary depending on experimental conditions, if conflicting results were collected for the same peptide, the corresponding peptide was indicated as a binding epitope. Since the peptides had different lengths, padding was applied to the longest peptide (16-mer). More specifically, in the case of 12-mer, padding was applied to the first and last two residues, and in the case of 15-mers, padding was applied to the last residue. The collected data was randomly split into training/validation and test sets in a 7 : 3 ratio to accurately evaluate model performance. Since the B cell and epitope binding was measured as general reactivity rather than a specific BCR sequence, the model was designed to comprehensively predict binding of peptides and a plurality of BCR sequences. More specifically, 48 human and 203 mouse immunoglobulin heavy chain variant (IGHV) alleles were collected from the IMGT database, and the human alleles were used as they were.

In the case of mouse alleles, one representative allele per superfamily was used to reduce computational burden. As a result, a total of 63 representative alleles including 48 human and 15 mouse alleles and corresponding base sequences were collected.

Prediction of B-cell epitopes was more difficult than prediction of T-cell epitopes, which was partially due to somatic hypermutation (SHM) that occurred in IGHV sequences. To describe the SHM, the previously calculated frequency of SHM per residue of the IGHV allelic superfamily was obtained. This ratio was calculated as the average SHM ratio per nucleotide position from 249,972 clones. For conversion to protein dimension, the SHM ratio per amino acid position was calculated as the average of three nucleotide sequences. If the upper series of the query allele was provided, the SHM ratio was used as it is, and if no upper series was provided, the SHM ratios of all alleles were used.

**Experimental Example 2: Construction of B-cell epitope prediction model**

To capture the unique local features of amino acid interactions between B-cell receptors (BCRs) based on a convolutional neural network (CNN) framework, a B-cell epitope prediction model (DeepNeo-BCR) was developed using the existing DeepNeo model architecture (Jeong Yeon Kim and others, DeepNeo: a webserver for predicting immunogenic neoantigens, Nucleic Acids Research, Volume 51, Issue W1, 5 July 2023, Pages W134-W140). To solve the variability of the BCR repertoire, sequence information was used included in > 370 million clones retrieved from 2,152 Ig sequencing samples (Yang, X. et al. Large-scale analysis of 2,152 Ig-seq datasets reveals key features of B cell biology and the antibody repertoire. Cell Rep. 35, 109110 (2021)).

The CNN architecture aimed to capture the preferential binding of BCRs and epitopes at amino acid levels. A two-dimensional matrix containing the interaction strengths between amino acids of the BCRs and the epitopes was generated based on previously calculated C$\alpha$-C$\alpha$ distances (Jha, A. N., Vishveshwara, S. & Banavar, J. R. Amino acid interaction preferences in proteins. Protein Sci. 19, 603-616 (2010)). In summary, the preferential binding matrix for each protein was generated considering the C$\alpha$-C$\alpha$ distance between amino acids, and a cutoff distance of 6.5 Å was used, excluding the nearest neighbors in the sequence. In addition, interatomic contacts between amino acid residues were also considered, and it was considered that residues i and j were in contact with each other if the atoms of the residues i and j were within a distance of 4.5 Å from the atoms of the residue j.

The preferential binding strength matrix between these amino acids was used to generate a CNN model input. In addition, SHM ratios with additional weights per residue were implemented to account for IGHV diversity. The SHM ratios calculated in previous studies were first normalized on a scale of 0 to 1 for each superfamily.

Then, the normalized SHM frequency per residue was reduced by a tenth part, using the SHM ratio as an auxiliary weight for amino acid preferential binding. The input value per matrix cell was defined as in Equation 1 below.

[Equation 1]

$$P_{ij} = \left( I_{ij} \right) \times (1 + SHM_i),$$

Here, i and j represented the positions of IGHV and a peptide sequence. $I_{ij}$ represented a preferential binding interaction between IGHV$_i$ and peptide$_j$, and the SHM frequency was given additional weight according to a value of IGHV$_i$. Since IEDB contained only information on B-cell binding and not the full sequences or genotypes of IGHV alleles, an input matrix was created for preferential amino acid binding between 63 representative IGHV alleles and peptides. For each allele, a CNN model was generated to generate 63 independent models that predicted the binding of the corresponding alleles and the peptides. The final prediction scores were calculated by applying linear regression to each CNN model. Since it was computationally difficult to predict all 63 models per peptide, linear regression models were constructed for purposes of using a full set (63 alleles), a human set (48 alleles), a human subset (25 alleles), and a mouse set (15 alleles).

The subset of human alleles was defined as representative alleles with sequence similarity of less than 80% as a result calculated by CD-HIT. The CNN model for individual alleles consisted of two convolutional layers, a fully connected layer, and a sigmoid layer. The convolutional layer was designed to capture the spatial interactions between IGHV and peptide sequences. Due to protein folding, contact residues in the BCR may not be contiguous, and to consider this feature, the first kernel contained the entire IGHV sequence. The convolutional layer extracted both contact and distal interaction features using a rectified linear activation function (ReLU) without pooling. 200 epochs were used to optimize the model, and various hyperparameter sets were used to ensure the best performance per CNN model. Specifically, combinations of various parameters, including learning rate (0.001, 0.01, 0.1), momentum (0.1, 0.5, 0.9), number of kernels in layers 1 and 2 (10, 30, 50), and normalized parameter (0.0001, 0.001, 0.01) were tested to confirm the optimal prediction accuracy. The model was tested with Keras on a TITAN XP GPU, and statistical analysis was performed with the Python scikit-learn package.

For model training, IgG binding peptides and non-binding peptides of fixed lengths (12-, 15-, and 16-mer) tested in humans or mice from IEDB were collected, and a ratio of binding peptides per peptide length was calculated (Table 1).

[Table 1]

| Peptide length | Entire peptide | Binding peptide (%) |
| --- | --- | --- |
| 12 mer | 107,640 | 77574 (72.1%) |
| 15 mer | 242,317 | 29477 (12.2%) |
| 16 mer | 87,438 | 39908 (45.6%) |
| Total | 437,395 | 146959 (33.6%) |

Since most of these datasets were based on testing on non-specific pools of the BCR repertoire, an ensemble model was designed by aggregating 63 CNN models, representing human or mouse immunoglobulin heavy chain variant (IGHV) alleles. In addition, somatic hypermutation (SHM) frequency per IGHV remnant was also incorporated into the model (FIG. 1A).

**Experimental Example 3: Prediction of linear B cell epitopes**

As described in Experimental Example 1 above, each peptide of the epitope was tested for all individual IGHV alleles, and linear regression was applied to the obtained predicted values to calculate the final predicted values. A B-cell epitope prediction model was constructed using four ensemble models such as all allele sets, all human alleles, all mouse alleles, or representative human alleles. As a result, the ensemble B-cell epitope prediction model showed better performance than models based on individual alleles (FIGS. 1B and 1C).

In addition, the performance of the ensemble B-cell epitope prediction model exhibited much better performance than not only conventional linear epitope prediction tools that analyzed fixed-length peptides, such as LBtope (Singh, H., Ansari, H. R. & Raghava, G. P. S. Improved method for linear B-cell epitope prediction using antigen's primary sequence. PLoS One 8, e62216 (2013)), LBEEP (Saravanan, V. & Gautham, N. Harnessing Computational Biology for Exact Linear B-Cell Epitope Prediction: A Novel Amino Acid Composition-Based Feature Descriptor. OMICS 19, 648-658 (2015).), and IgPred (Gupta, S., Ansari, H. R., Gautam, A., Raghava, G. P. S. & Consortium, O. S. D. D. Identification of B-cell epitopes in an antigen for inducing specific class of antibodies. Biol. Direct 8, 27 (2013).), but also a BepiBred-3.0 model working on both linear and conformational epitopes (Clifford, J. N. et al. BepiPred-3.0: Improved B-cell epitope prediction using protein language models. Protein Sci. 31, e4497 (2022).) (FIG. 1D). For additional benchmarking, 116 new IEDB data items that were not included in model training or testing were collected. Even in the corresponding dataset, the B-cell epitope prediction model had better performance than all other conventional methods (FIG. 1E).

**Example 1: Validation using SARS-CoV-2 vaccination data**

B cells with BCR sequences that recognized immunogenic epitopes were subjected to clonal expansion, and B cell clonal expansion in response to SARS-CoV-2 vaccination has been reported in several studies. Accordingly, BCR sequences from vaccinated individuals were tested to determine whether BCRs with clonal expansion during COVID-19 vaccination had high B cell epitope prediction scores for SARS-CoV-2 epitopes. First, large-scale BCR sequencing data were collected for nine subjects who received a Pfizer SARS-CoV-2 mRNA vaccine. IGHV sequences were extracted and clustered into clones, which were divided into a large (> mean read fraction) clone group and a small (< mean read fraction) clone group (FIG. 2A). The affinity of each IGHV sequence was assessed using a B-cell epitope prediction model for all translated vaccine sequences. As a result, the mean B cell epitope prediction score of the large clones was higher than that of the small clones (FIG. 2B). In addition, enrichment of high-affinity clones for a large-scale group (> mean B cell epitope prediction score) was calculated per sample, and as a result, an average odd ratio was 1.5 (FIG. 2C), suggesting that IGHV sequences with high predicted affinity for vaccine sequences were prone to clonal expansion.

To more accurately identify BCR clones and directly measure clonal expansion, single-cell BCR sequencing data were generated for 24 blood samples before and after Pfizer SARS-CoV-2 vaccination (Table 2).

[Table 2]

| Feature | | N = 33 | % |
|---|---|---|---|
| Sex | | | |
| | Male | 10 | 42 |
| | Female | 14 | 58 |
| Age | | | |
| | Median | 36.5 | |
| | Range | 29-44.3 | |
| Vaccine | | | |
| | Pfizer | 24 | 100 |

Individual cells of each sample were clustered into clones based on similarity in BCR sequences, and clonal expansion was determined by comparing ratios of matching BCR clones before and after vaccination (FIG. 2D). Epitope binding prediction was performed for the BCR sequences within the identified clones. As a result, clones with high affinity to the vaccine sequence tended to undergo clonal expansion during vaccination more than clones with low affinity (FIGS. 2E and 2F). Furthermore, analysis for post-vaccination clones was performed repeatedly on large BCR data, and as a result, like previous study results, larger post-vaccination clones (> mean read fraction) were more likely to have higher affinity for the vaccine sequence predicted by the B-cell epitope prediction model (FIG. 2G). A high level of matching degree was also found among clonally expanded BCRs (FIG. 2H). In addition, the matched single-cell transcriptome data was investigated, and then a total of 76,843 cells were collected, and among them, 4,146 cells were mature B cells and 6,185 cells were naive B cells (FIG. 2I). Mature B cells had higher BCR scores than naive B cells (FIG. 2J), leading to an overrepresentation of high-affinity BCRs in mature B cells compared to naive B cells (FIG. 2K).

In summary, the consistency of results obtained from two different datasets validated the validity of the B-cell epitope prediction model. BCR clones with high B cell epitope scores, indicating stronger predicted affinity to SARS-CoV-2 vaccine sequences, were shown to have an increased propensity for clonal expansion during COVID-19 vaccination.

**Example 2: Mouse vaccination data analysis**

DeepNeo-TCR, an algorithm for evaluating T cell responses to a given peptide-MHC complex developed previously (Kim, J. Y. et al. MHC II immunogenicity shapes the neoepitope landscape in human tumors. Nat. Genet. 55, 221-231 (2023).) was used to identify TCR(+) epitopes in three mouse cancer models (EMT6 breast cancer, LLC1 lung cancer, and B16F10 melanoma). Based on the prediction, 46 synthesized long peptides were prepared and administered three times to mice, and spleen cells were collected and IFNγ secretion was measured using the ELISpot assay. To ensure reproducibility, each peptide was tested in three mice, and each mouse was analyzed twice, resulting in a total of six measurements per peptide. As a result, a total of 276 measurements were performed for 46 candidate neoantigens. For most neoantigens, IFNγ signals were stronger than in a negative control group.

Here, this dataset was reanalyzed by executing a B-cell epitope prediction model on all potential neoepitopes within 44 synthesized long peptides after filtering two outliers. Since wild-type sequences were used for all neoantigens, a wild-type score was subtracted from a mutant score to obtain differential B-cell epitope prediction scores for each neoepitope. The maximum differential B-cell epitope prediction score of all potential neoepitopes in the vaccines was obtained, and 44 vaccine compositions were divided into two groups according to this score: TCR(+)BCR(high) represented vaccines with a higher-than-average B-cell epitope prediction score, and TCR(+)BCR(low) represented vaccines with a lower-than-average B-cell epitope prediction score (Table 3).

[Table 3]

| Cancer cell line | Mutation | Peptide sequence | IFN analysis | B-cell epitope prediction model group |
|---|---|---|---|---|
| B6F10 | Anln-p.E580G | GELDVEKSQGEMDQVG AENSEEQE | 1,1,24,2,7,7 | low |
| B6F10 | Cpsf6-p.D519G | SRDRHGDYYRERSRER ERHRDRDR | 11,19,1,2,3,6 | low |
| B6F10 | Dnajb12-p.P54T | LNQKTQSTGDHPQPTDT THTTTKK | 0,0,23,7,9,9 | high |
| B6F10 | Hjurp-p.Q402P | PRMLPSPVEKWYREIKI KFDKLHQ | 0,0,22,23,8,9 | low |
| B6F10 | Hnrnpk-p.R326G | PPPPRGGDLMAYDRGG RPGDRYDG | 0,10,17,1,7,7 | high |
| B6F10 | Macroh2a1-p.P107L | TIASGGVLLNIHPELLAK KRGSKG | 106,19,36,6,6,72 | low |
| B6F10 | Map2k2-p.A29G | TINPTIAEGPSPTSEGGS EANLVD | 16,19,1,1,48,61 | low |
| B6F10 | Nacc1-p.A434G | RKPLDSRVLHAVKYYC QNFGPNFK | 154,160,3,4,58,60 | high |
| B6F10 | Oaz1-p.R24G | REKEGDKGSATLHASR TMPLLSQH | 10,1,21,2,2,2 | low |
| B6F10 | Polr2b-p.I903S | RRYTKRDCSTFLRTSET GSVDQVM | 17,19,1,3,51,6 | low |
| B6F10 | Rbbp6-p.P1232H | RETGKKIGNAENASTTK EHSEKLE | 0,10,13,1,6,7 | low |
| B6F10 | Rp112-p.I82N | SALINKALKEPPRDRKK QKNIKHS | 17,3,40,4,62,66 | high |
| B6F10 | Sbno1-p.K720N | TREAKKARNVGGLTGS SSDDSGSE | 22,2,3,3,3,8 | low |
| B6F10 | Spen-p.E1113D | AGLGELTHGSVDTQET QPKKAIPSK | 142,19,2,3,47,93 | low |
| EMT6 | Aatf-p.T442I | DFYHQLLRELIERKISSL DPNDQVAMGRQ | 26,32,42,56,57,78 | high |
| EMT6 | Atp6v1a-p.Q441E | DKKLAERKHFPSVNWLI SYSKYMR | 10,13,14,32,34,9 | low |
| EMT6 | Atp6v1f-p.N99D | SKEHPYDAAKDSILRRA KGMFTAE | 12,12,13,4,9,9 | low |

(continued)

| Cancer cell line | Mutation | Peptide sequence | IFN analysis | B-cell epitope prediction model group |
|---|---|---|---|---|
| EMT6 | Cdc42-p.D63E | PYTLGLFDTAGQEEYDR LRPLSYPQTDV | 0,0,1,1,1,3 | high |
| EMT6 | Cltc-p.Y417F | PGQTSPLLQFFGILLDQG QLNKYE | 40,53,63,66,77,81 | high |
| EMT6 | Dnmt1-p.D576G | FVVSQVESYDEAKDGD ETPIFLSPCMRAL | 1,2,2,2,7,8 | high |
| EMT6 | Dync1h1-p.N2529S | TVPLPTAPSVPIIDYEVSI SGEWS | 13,16,16,20,22,29 | low |
| EMT6 | Hjurp-p.R157G | REASGDPRQRQPAVPG NTLETDLR | 10,2,2,3,4,7 | high |
| EMT6 | Iars-p.V178G | KQLYDKGLVYRGVKG MPFSTACGTPLSNF | 11,16,16,7,7,8 | high |
| EMT6 | Med29-p.G152D | PTATKPDAVQPDSLPYP QYLAVIK | 10,10,12,4,6,7 | high |
| EMT6 | Msn-p.F84L | AQDVRKESPLLFKFRAK LYPEDVS | 0,1,1,1,3,4 | low |
| EMT6 | Nxf1-p.S598Y | DNNWDYTRYAQAFTLL KAKGEIPE | 24,24,28,48,57,61 | low |
| EMT6 | Pdhb-p.H138Q | SAGLQPVPIVFRGPNGA SAGVAAQ | 0,1,2,3,3,4 | high |
| EMT6 | Phip-p.N671D | RRSGEAGVSNASRVDR GSVSSTSEVHS | 0,1,1,4,4,6 | low |
| EMT6 | Ras12-9-p.F162L | DISARSNYNFEKPFLWL ARKLIGDPNLEF | 0,10,1,2,3,6 | high |
| EMT6 | Sf3b1-p.H886R | METIEKIMGNLGAADID RKLEEQL | 11,13,14,17,18,24 | low |
| EMT6 | Ssrp1-p.K623E | KAMKEYEGGRGDSSER DKSKKKKKVKAKM | 0,1,3,3,6,7 | low |
| EMT6 | Stt3b-p.G804A | KQKYLSKKTTKRKRAY VKNKLVFKKGKKT | 0,0,0,2,2,2 | low |
| EMT6 | Yae1d1-p.E151K | DMDLCHVVPSDQAKGD SNAEINTDSDS | 10,13,17,3,9,9 | low |
| LLC1 | Chd8-p.K445N | PASCAPHTAGNTGMEE NRRLEHQKK | 0,19,1,1,1,4 | low |

(continued)

| Cancer cell line | Mutation | Peptide sequence | IFN analysis | B-cell epitope prediction model group |
|---|---|---|---|---|
| LLC1 | Ddb1-p.I959F | RDFNPNWMSAVEFLDD DNFLGAEN | 12,1,20,3,7,9 | high |
| LLC1 | EifSb-p.S815T | KDPRTFVTLVPTSAHTG DGMGSLI | 108,136,19,43,51,9 | low |
| LLC1 | Ewsr1-p.T97A | YSQPVQGYGTGAYDST TATVTTTQ | 11,1,2,36,3,8 | high |
| LLC1 | Mllt3-p.M352T | SETSEKKKSTLPPFDDIV DPNDSD | 1,21,27,2,4,8 | high |
| LLC1 | Myh9-p.W26C | DKYLYVDKNFINNPLA QADCAAKK | 14,16,56,61,72,78 | high |
| LLC1 | Prpf8-p.K1955N | KVILNPDKTTVTEPHHI WPTLTDE | 1,1,3,7,7,8 | high |
| LLC1 | Psmb6-p.A60G | DSRTTTGSYIGNRVTDK LTPIHDH | 0,1,2,3,3,7 | low |
| LLC1 | Rlim-p.I312V | SDTGSNSESSGSGQRPP TVVLDLQ | 10,11,1,3,3,7 | low |
| LLC1 | Rrs1-p.G314W | SQKGKRKWGRQGPSGK RKGGPPGQ | 0,2,4,4,6,6 | high |
| LLC1 | Tns3-p.L1051M | VNSHGTSPAPGTPLMTT GAADNGFLPH | 1,2,2,3,4,8 | high |

As a result, the TCR(+)BCR(high) vaccines showed a stronger IFNγ signal than the TCR(+)BCR(low) vaccines (FIG. 3), suggesting that B cell reactivity to T cell immunogenic neoantigens may induce a stronger immune response. A similar pattern was observed even when the same analysis was repeated considering only mutation scores (FIG. 6). These results indicated that neoantigens that stimulated not only B cells but also T cells may induce a more potent immune response than neoantigens that did not bind to B cells.

**Example 3: Human clinical trial data analysis**

To determine the effect of neoantigen vaccines on B cell reactivity in a clinical environment, data of 12 clinical trials were collected from 125 patients administered with 1,766 vaccines in the form of mRNA constructs, synthesized long peptides, or peptide-loaded DCs (Table 4).

[Table 4]

| Source | Issued year | Sample number | Peptide number | Cancer type | Vaccine type | HLA genotype | PMID |
|---|---|---|---|---|---|---|---|
| Rojas | 2023 | 16 | 232 | PDAC | mRNA | o | 37165196 |
| Awad | 2022 | 9 | 176 | NSCLC | Peptide | x | 36027916 |
| Sahin | 2017 | 13 | 124 | SKCM | mRNA | x | 28678784 |
| Ott_2017 | 2017 | 6 | 98 | SKCM | Peptide | o | 28678778 |

(continued)

| Source | Issued year | Sample number | Peptide number | Cancer type | Vaccine type | HLA genotype | PMID |
|---|---|---|---|---|---|---|---|
| Keskin | 2019 | 8 | 103 | GBM | Peptide | x | 30568305 |
| Chen | 2021 | 6 | 74 | PDAC | Peptide | o | 34484187 |
| Fang | 2020 | 11 | 163 | SKCM,COAD, NSCLC,PDAC ,OV,BTC | Peptide | o | 32439700 |
| Cai | 2021 | 6 | 60 | HCC | Peptide | x | 34903219 |
| Ott_2020 | 2020 | 34 | 553 | SKCM,NSCL C,BLCA | Peptide | x | 33064988 |
| Hu | 2021 | 2 | 27 | SKCM | Peptide | x | 33479501 |
| Cafri | 2020 | 4 | 76 | GI | mRNA | x | 33016924 |
| Peng | 2022 | 10 | 80 | HCC | Dendritic cell | o | 35476700 |
| Total | | 125 | 1766 | | | | |

Each patient was treated with 5 to 30 vaccines producing 14 to 52-mer peptides. All potential neoepitopes of each vaccine were analyzed with DeepNeo-TCR and a B-cell epitope prediction model (DeepNeo-BCR) to identify TCR(+) and BCR(+) epitopes (FIG. 4A). When classified by a T cell response measured by IFN$\gamma$ secretion, the IFN$\gamma$(+) vaccines had a much greater ratio of TCR(+) epitopes (FIG. 4B). This suggested that considering only MHC binding in a neoantigen design may not guarantee a strong T cell response. Crucially, vaccines containing more TCR(+)BCR(+) epitopes were more likely to induce IFN$\gamma$ release than vaccines containing fewer TCR(+)BCR(+) epitopes (FIG. 4C), suggesting that B cell-reactive neoepitopes also played an important role in promoting T cell responses. For example, for vaccines containing 20% or more of TCR(+)BCR(+) epitopes, the probability of IFN$\gamma$ stimulation was 1.75 times higher than that of vaccines containing fewer these neoepitopes (P = $3.96 \times 10^{-3}$) (left in FIG. 4D). This odd ratio was further increased in vaccines with 30% or more of TCR(+)BCR(+) epitopes (OR = 2.41, P= $7.34 \times 10^{-5}$) (right in FIG. 4D). Activated B cells may potentially interact with CD4+ T cells that recognized different antigens or different epitopes of the same antigen. Therefore, the B cell epitopes may enhance not only the homologous vaccine in which the corresponding epitopes were present, but also other vaccines with different epitope compositions introduced simultaneously into the same patient. To test this possibility, overall immune responses to vaccination of each patient measured as the ratio of IFN$\gamma$(+) vaccines were evaluated in relation to overall B cell reactivity to all delivered neoantigens.

First, the patient-level B-cell reactivity of the vaccines was confirmed using the maximum B-cell epitope prediction score among all neoepitopes per patient. This index showed a strong positive correlation with the patient-level immune response (R = 0.32; P = $1.9 \times 10^{-4}$) (FIG. 4E). For example, a patient classified as 'Sahin P04' was vaccinated with 'vac1', a vaccine with a high B cell epitope prediction score, and showed an IFN$\gamma$(+) response to 8 out of 10 vaccines (top in FIG. 4F). 'Chen P04' responded to 3 out of 13 vaccines having no BCR(+) epitope in all vaccines (bottom in FIG. 4F). In contrast, the DeepNeo-TCR scores did not show such a correlation with patient-level immune responses (FIG. 7A). This pattern was not found in other B cell epitope prediction tools, LBtope, LBEEP, IgPred, and BepiPred3.0 (FIG. 8).

Next, the percentage of BCR(+) epitopes for all vaccines per patient was used as a measurement value of total B cell reactivity to the vaccines. This measurement value also showed a positive correlation with the ratio of IFN$\gamma$(+) vaccines (R = 0.25; P = $9.6 \times 10^{-3}$) (FIG. 7B). For example, when 70% or more of IFN$\gamma$(+) vaccines was considered as a sufficient immune response, 20% or more of BCR(+) epitopes was included to 4.72-fold increase the probability of inducing a patient-level response to vaccination (P = $4.11 \times 10^{-3}$; FIG. 4G). When the same analysis was repeated with other B cell epitope prediction tools, no contribution of B cell neoepitopes to the vaccine response of the patient was observed (FIG. 9).

When these results were combined, it was confirmed that patients vaccinated with a vaccine formulation favorable for B cell reactivity were more likely to exhibit robust T cell responses, and that the B-cell epitope prediction model may reliably predict B cell reactivity when considering the overall effects of all vaccines administered as well as individual neoantigens.

### Example 4: Tumor tissue data analysis

Next, it was confirmed whether B cell neoepitopes could affect the intratumoral BCR repertoire. To this end, transcriptomes matching the exomes of 4,435 TCGA samples were analyzed. The B-cell neoepitope load was measured by applying a B-cell epitope prediction model to the somatic mutations in each sample. The characteristics of the BCR repertoire were calculated in terms of clonality and entropy. For comparison, the T-cell neoepitope load was calculated using DeepNeo-TCR and the clonality and entropy of the T cell receptor (TCR) repertoire were determined using transcriptome data.

First, it was observed that individual tumors with a high BCR(+) epitope load exhibited higher BCR clonality and lower BCR entropy (FIG. 10). Then, each sample was classified into the corresponding tumor type and the average value for each tumor type was derived. As a result, a positive correlation between BCR(+) epitope load and BCR clonality and a negative correlation between BCR(+) epitope load and BCR entropy became more prominent (FIG. 5A). Moreover, these correlations were most pronounced between BCR(+) epitope load and BCR characteristics. The BCR clonality and entropy were less correlated with the TCR(+) epitope load than with the BCR(+) epitope load (FIG. 5B). Similarly, the TCR clonality showed a weaker correlation with the BCR(+) epitope load than the TCR(+) epitope load (FIG. 5B). However, unlike the BCR entropy, the TCR entropy did not show a negative correlation with the TCR(+) epitope load. These results showed that the B-cell epitope prediction model may identify properties of B-cell neoepitopes even within the local tumor environment.

The above-described method may be provided by a computer program stored in a computer-readable recording medium to be executed on a computer. The medium may continuously store or temporarily store a computer-executable program for execution or download. Further, the medium may be various recording means or storage means in which single hardware or several hardware is coupled, but is not limited to a medium directly connected to any computer system and may also be distributed on a network. Examples of the medium may include magnetic media such as hard disks, floppy disks, and magnetic tape, optical media such as CD-ROM and DVD, magneto optical media such as floptical disks, ROM, RAM, flash memories, etc. and may be configured to store program instructions. Further, examples of other media may also include recording media or storage media managed in app stores for distributing applications, or sites and servers for supplying and distributing other various software, etc.

The above-described methods, operations or techniques may be implemented by various means. For example, these techniques may be implemented in hardware, firmware, software, or a combination thereof. Whether these methods are implemented as hardware or software depends on design requirements imposed on a specific application and the overall system. Those skilled in the art may also implement functions described in various ways for each specific application, but such implementations should not be construed without departing from the scope of the present disclosure.

In the hardware implementation, processing units used to perform the techniques may also be implemented within one or more ASICs, DSPs, digital signal processing devices (DSPDs), programmable logic devices (PLDs), field programmable gate arrays (FPGAs), processors, controllers, microcontrollers, microprocessors, electronic devices, other electronic units designed to perform the functions described herein, computers, or combinations thereof.

When implemented in software, the above-described methods may be stored on computer-readable media or also transmitted via computer-readable media with one or more instructions or codes. The computer-readable media include both computer storage media and communication media, including any media that facilitates transmission of computer programs from one place to the other place. The storage media may be any available media that may be accessed by computers. As a non-limiting example, these computer-readable media may include RAM, ROM, EEPROM, CD-ROM or other optical disk storage, magnetic disk storage or other magnetic storage devices, or any other media that may be used to carry or store desired program codes in the form of instructions or data structures and may be accessed by computers. In addition, any access is properly referred to as a computer-readable medium.

As described above, although the embodiments have been described by the restricted drawings, various technical modifications and variations can be applied on the basis of the embodiments by those skilled in the art. For example, even if the described techniques are performed in a different order from the described method, and/or components described above are coupled or combined in a different form from the described method, or replaced or substituted by other components or equivalents, an appropriate result can be achieved.

Therefore, other implementations, other embodiments, and equivalents to the appended claims fall within the scope of the claims to be described below.

## Claims

1.    A method for designing a personalized cancer vaccine, using a B cell-reactive neoantigen, the method comprising the steps of:

receiving a peptide sequence extracted from cancer tissue and allelic sequence data of B cell receptors;
calculating preferential binding strength between amino acids in the peptide sequence and the allelic sequences of the B cell receptors; and
calculating B cell epitope prediction scores by inputting the preferential binding strength between the amino acids to a trained neural network model.

**2.** The method of claim 1, wherein the step of calculating the preferential binding strength between amino acids in the peptide sequence and the allelic sequences of the B cell receptors includes
a step of generating a matrix including Cα-Cα distance information between the amino acids and interatomic contact information between amino acid residues in the peptide sequence and the allelic sequences of the B cell receptors.

**3.** The method of claim 2, wherein the matrix includes somatic hypermutation (SHM) frequency data per residue of allelic superfamily of the B cell receptors.

**4.** The method of claim 3, wherein cell values constituting the matrix are defined by the following Equation 1:

[Equation 1]

$$P_{ij} = \left(I_{ij}\right) \times \left(1 + SHM_i\right),$$

wherein i and j represent positions of an immunoglobulin heavy chain variant (IGHV) of the B cell receptor and the peptide sequence, and $I_{ij}$ represents a preferential binding interaction between IGHV$_i$ and peptide$_j$.

**5.** The method of claim 1, wherein the neural network model is a convolutional neural network (CNN) model, and the CNN model includes a convolutional layer that detects spatial interactions between the immunoglobulin heavy chain variant (IGHV) of the B cell receptor and the peptide sequence.

**6.** The method of claim 5, wherein the CNN model consists of a plurality of convolutional layers, a fully connected layer, and a sigmoid layer.

**7.** The method of claim 5, wherein a kernel of the CNN model includes an entire amino acid sequence of the immunoglobulin heavy chain variant (IGHV) of the B cell receptor.

**8.** The method of claim 5, wherein the convolutional layer extracts features of contact and distal interactions using a pooling-free rectified linear unit (ReLU) function.

**9.** The method of claim 5, wherein the neural network model is an ensemble model that aggregates the plurality of CNN models.

**10.** The method of claim 1, wherein the step of calculating the B cell epitope prediction scores includes
a step of applying linear regression to output values of the neural network model.

**11.** The method of claim 1, wherein a neoantigen with the calculated B cell epitope prediction scores higher than an average B cell epitope prediction score is determined to have a high ability to induce an immune response.

**12.** A computer program stored in a computer-readable recording medium for executing the method of any one of claims 1 to 10 on a computer in combination with hardware.

**13.** A computer-readable recording medium recording programs for executing the method of any one of claims 1 to 10 on a computer.

[FIG 1A]

IGHV sequence

Epitope

AA preferential binding strength

SHM frequency

V allele 1
V allele 2
V allele 3
V allele 63

$$y = \beta_0 + \beta_1 x_1 + \beta_2 x_2 + \cdots + \beta_i x_i$$

EP 4 765 132 A1

16

【FIG 1B】

【FIG 1C】

[FIG 1D]

Model construction data

[FIG 1E]

Common test data

【FIG 2A】

Bulk BCR-seq clustering

: Reads

Clone 1
Clone 2
Clone 3
Clone 4

【FIG 2B】

Density

Mean predicted affinity score

Large clone
Small clone

【FIG 2C】

Odd ratio : mean, 95% CI

【FIG 2D】

【FIG 2E】

【FIG 2F】

## High affinity clones

- Post vaccine fraction
- Pre vaccine fraction

## Low affinicity clones

- Post vaccine fraction
- Pre vaccine fraction

【FIG 2G】

## Odd ratio : mean, 95% CI

【FIG 2H】

【FIG 2I】

【FIG 2J】

【FIG 2K】

【FIG 3A】

Combined

【FIG 3B】

【FIG 3C】

- B16F10 M1
- B16F10 M10
- B16F10 M11
- B16F10 M12
- B16F10 M2
- B16F10 M3
- B16F10 M4
- B16F10 M5
- B16F10 M6
- B16F10 M7
- B16F10 M8
- B16F10 M9
- EMT6 M1
- EMT6 M10
- EMT6 M11
- EMT6 M12
- EMT6 M2
- EMT6 M3
- EMT6 M4
- EMT6 M5
- EMT6 M6
- EMT6 M7
- EMT6 M8
- EMT6 M9
- LLC1 M1
- LLC1 M10
- LLC1 M11
- LLC1 M12
- LLC1 M2
- LLC1 M3
- LLC1 M4
- LLC1 M5
- LLC1 M6
- LLC1 M7
- LLC1 M8
- LLC1 M9

TCR(+)BCR(high) vaccines

TCR(+)BCR(low) vaccines

IFNγ count

150

100

50

0

【FIG 4A】

【FIG 4B】

【FIG 4C】

【FIG 4D】

FIG 4E]

**% IFNγ(+) vaccines**

R = 0.32
P = 1.9e-4

Sahin P04

Chen P04

Patient-level B cell epitope score
(Max DeepNeo-BCR score per patient)

【FIG 4G】

|  | IFNγ(+) vaccines > 70% | IFNγ(+) vaccines < 70% |
|---|---|---|
| BCR(+) > 20% per patient | 26 | 55 |
| BCR(+) < 20% per patient | 4 | 40 |

OR = 4.72, P = 4.11e-3

【FIG 5A】

【FIG 5B】

【FIG 6A】

【FIG 6B】

【FIG 6C】

【FIG 7】

【FIG 8】

【FIG 9】

LBEEP

|  | IFNY (+) vaccine>70% | IFNY (+) vaccine<70% |
|---|---|---|
| BCR(+) > 20% | 23 | 57 |
| BCR(+) < 20% | 7 | 38 |

OR = 2.19, P = 0.13

BepiPred3.0

|  | IFNY (+) vaccine>70% | IFNY (+) vaccine<70% |
|---|---|---|
| BCR(+) > 20% | 20 | 73 |
| BCR(+) < 20% | 10 | 22 |

OR = 0.60, P = 0.34

LBtope

|  | IFNY (+) vaccine>70% | IFNY (+) vaccine<70% |
|---|---|---|
| BCR(+) > 20% | 15 | 56 |
| BCR(+) < 20% | 15 | 39 |

OR = 0.696, P = 0.406

IgPred

|  | IFNY (+) vaccine>70% | IFNY (+) vaccine<70% |
|---|---|---|
| BCR(+) > 20% | 14 | 62 |
| BCR(+) < 20% | 16 | 33 |

OR = 0.46, P = 0.087

【FIG 10】

## INTERNATIONAL SEARCH REPORT

| International application No. |
|---|
| **PCT/KR2024/011714** |

### A. CLASSIFICATION OF SUBJECT MATTER

**G16B 40/20**(2019.01)i; **G16B 30/00**(2019.01)i; **G16B 15/30**(2019.01)i; **G16B 20/20**(2019.01)i; **G16B 20/50**(2019.01)i; **G06N 3/0464**(2023.01)i; **G06N 3/09**(2023.01)i; **G06N 20/20**(2019.01)i

According to International Patent Classification (IPC) or to both national classification and IPC

### B. FIELDS SEARCHED

Minimum documentation searched (classification system followed by classification symbols)

G16B 40/20(2019.01); G06F 19/24(2011.01); G06F 19/26(2011.01); G16B 15/30(2019.01); G16B 20/00(2019.01); G16B 20/20(2019.01); G16B 20/30(2019.01); G16B 30/10(2019.01); G16B 35/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 암 조직(cancer tissue), 추출(extraction), 펩타이드 서열(peptide sequence), B세포 수용체(B-cell receptor), 대립 유전자(allele gene), 서열 데이터(sequence data), 결합 강도(binding strength), 신경망 모델(neural network model), 예측 점수(prediction score), 계산(calculation), B세포 반응성 신생항원(B-cell reactive neoantigen), 암백신(cancer vaccine), 설계(design)

### C. DOCUMENTS CONSIDERED TO BE RELEVANT

| Category* | Citation of document, with indication, where appropriate, of the relevant passages | Relevant to claim No. |
|---|---|---|
| A | KR 10-2159921 B1 (THERAGEN BIO CO., LTD.) 25 September 2020 (2020-09-25)<br>See abstract, claim 1, and paragraphs [0006], [0008] and [0023]. | 1-13 |
| A | KR 10-2278727 B1 (THERAGEN BIO CO., LTD.) 19 July 2021 (2021-07-19)<br>See entire document. | 1-13 |
| A | KR 10-2184720 B1 (KOREA ADVANCED INSTITUTE OF SCIENCE AND TECHNOLOGY) 30 November 2020 (2020-11-30)<br>See entire document. | 1-13 |
| A | KR 10-2018-0052959 A (KOREA INSTITUTE OF SCIENCE & TECHNOLOGY INFORMATION) 21 May 2018 (2018-05-21)<br>See entire document. | 1-13 |

☑ Further documents are listed in the continuation of Box C.  ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **18 November 2024** | **20 November 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/011714**

| C. | DOCUMENTS CONSIDERED TO BE RELEVANT | |
|---|---|---|
| Category* | Citation of document, with indication, where appropriate, of the relevant passages | Relevant to claim No. |
| A | KR 10-2475794 B1 (THERAGEN BIO CO., LTD.) 08 December 2022 (2022-12-08)<br>See entire document. | 1-13 |

Form PCT/ISA/210 (second sheet) (July 2022)

**INTERNATIONAL SEARCH REPORT**
Information on patent family members

International application No.

**PCT/KR2024/011714**

| Patent document cited in search report | | | Publication date (day/month/year) | Patent family member(s) | | | Publication date (day/month/year) |
|---|---|---|---|---|---|---|---|
| KR | 10-2159921 | B1 | 25 September 2020 | CN | 115298739 | A | 04 November 2022 |
| | | | | EP | 4131275 | A1 | 08 February 2023 |
| | | | | JP | 2023-518725 | A | 08 May 2023 |
| | | | | US | 2024-0203522 | A1 | 20 June 2024 |
| | | | | WO | 2021-194057 | A1 | 30 September 2021 |
| KR | 10-2278727 | B1 | 19 July 2021 | | None | | |
| KR | 10-2184720 | B1 | 30 November 2020 | WO | 2021-071182 | A1 | 15 April 2021 |
| KR | 10-2018-0052959 | A | 21 May 2018 | KR | 10-1925040 | B1 | 04 December 2018 |
| KR | 10-2475794 | B1 | 08 December 2022 | CN | 117672348 | A | 08 March 2024 |
| | | | | EP | 4328919 | A1 | 28 February 2024 |
| | | | | JP | 2024-031787 | A | 07 March 2024 |
| | | | | US | 2024-0071564 | A1 | 29 February 2024 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JEONG YEON KIM**. DeepNeo: a webserver for predicting immunogenic neoantigens. *Nucleic Acids Research*, 05 July 2023, vol. 51 (W1), W134-W140
- **YANG, X. et al.** Large-scale analysis of 2,152 Ig-seq datasets reveals key features of B cell biology and the antibody repertoire. *Cell Rep.*, 2021, vol. 35, 109110
- **JHA, A. N.** ; **VISHVESHWARA, S.** ; **BANAVAR, J. R.** Amino acid interaction preferences in proteins. *Protein Sci.*, 2010, vol. 19, 603-616
- **SINGH, H.** ; **ANSARI, H. R** ; **RAGHAVA, G. P. S.** Improved method for linear B-cell epitope prediction using antigen's primary sequence.. *PLoS One*, 2013, vol. 8, e62216
- **SARAVANAN, V.** ; **GAUTHAM, N**. Harnessing Computational Biology for Exact Linear B-Cell Epitope Prediction: A Novel Amino Acid Composition-Based Feature Descriptor. *OMICS*, 2015, vol. 19, 648-658
- **GUPTA, S** ; **ANSARI, H. R.** ; **GAUTAM, A** ; **RAGHAVA, G. P. S** ; **CONSORTIUM, O. S. D. D**. Identification of B-cell epitopes in an antigen for inducing specific class of antibodies. *Biol. Direct*, 2013, vol. 8, 27
- **CLIFFORD, J. N. et al.** BepiPred-3.0: Improved B-cell epitope prediction using protein language models. *Protein Sci.*, 2022, vol. 31, e4497
- **KIM, J. Y. et al.** MHC II immunogenicity shapes the neoepitope landscape in human tumors. *Nat. Genet.*, 2023, vol. 55, 221-231